# EUROPÄISCHE PATENTANMELDUNG

(11) **EP 2 085 094 A2**
(43) Veröffentlichungstag der Anmeldung: **05.08.2009**
(21) Anmeldenummer: 09160281.3
(22) Anmeldetag: 04.07.2003
(51) Int. Cl.: A61K 38/00, A61P 37/06, A61K 31/66

(54) **Verwendung von Inhibitoren der Alanyl-Aminopeptidasen und diese umfassende pharmazeutischen Zubereitungen**

(30) Priorität: 05.07.2002 DE 10230381
(62) Teilanmeldung aus: 03762633.0
(71) Anmelder: IMTM GmbH, 39120 Magdeburg (DE)
(72) Erfinder: Ansorge, Siegfried, 39291 Hohenwarthe (DE); Tadje, Janine, 39118 Magdeburg (DE); Lendeckel, Uwe, 39120 Magdeburg (DE)
(74) Vertreter: Koepe & Partner

(57) **Zusammenfassung**

Die vorliegende Erfindung betrifft die Verwendung von einem Inhibitor oder mehreren Inhibitoren von Alanyl-Aminopeptidasen und/oder von Enzymen gleicher Substratspezifität zur Induktion der Produktion von TGF-β1 und Expression von TGF-β1 in und/oder auf Trcg-Zellen und die Verwendung zur Vorbeugung und/oder Behandlung von Autoimmunerkrankungen, Allergien, Arteriosklerose und zur Unterdrückung von Transplantat-Abstossungen. Weiter betrifft die Erfindung Verwendungen bei der zusätzlich Peptidfragmente von pathogenen Autoantigcncn oder synthetische Analoga und/oder spezifische antigcnc Komponenten pathogener Mikroorganismen eingesetzt werden.

## Beschreibung

Die vorliegende Erfindung betrifft die Verwendung von einem Inhibitor oder mehreren Inhibitoren von Alanyl-Aminopeptidasen und/oder von Enzymen gleicher Substratspezifität zur Induktion der Produktion von TGF-β1 und Expression von TGF-β1 in und/oder auf Treg-Zellen und die Verwendung zur Vorbeugung und/oder Behandlung von Autoimmunerkrankungen, Allergien, Arteriosklerose und zur Unterdrückung von Transplantat-Abstossungen. Weiter betrifft die Erfindung Verwendungen bei der zusätzlich Peptidfragmente von pathogenen Autoantigenen oder synthetische Analoga und/oder spezifische antigene Komponenten pathogener Mikroorganismen eingesetzt werden. Die gemeinsame Applikation von Inhibitoren gegen oben genannte Enzyme mit krankheitsspezifischen Antigenen verstärkt die gerichtete Wirkung der Inhibitoren gegen pathogene T-Zell-Klone und ist zur spezifischen Therapie immunologisch bedingter Erkrankungen geeignet.

Es ist bereits bekannt, daß bei Erkrankungen mit Autoimmunpathogenese wie beispielsweise Typ I Diabetes mellitus oder Multiple Sklerose eine Aktivierung und Proliferation von autoreaktiven, d.h. gegen körpereigene Antigene gerichtete Immunzellen, insbesondere von autoreaktiven T-Lymphozyten, dem Krankheitsprozess zugrunde liegen bzw. diesen ausmachen. Ähnliche Mechanismen kommen bei der Entstehung von Abstossungsepisoden nach Organtransplantation mit dem Unterschied zum Tragen, dass hier nicht primär "Autoantigene", sondern "Fremdantigene" aus dem Spenderorgan für die Entwicklung der fatalen Immunantwort verantwortlich sind. In beiden Fällen, d.h. sowohl bei Autoimmunerkrankungen als auch bei Abstossungsreaktionen, kommt es zu einem unerwünschten Bruch der "Toleranz" des Immunsystems gegen die körpereigenen oder vom Transplantat herrührenden Antigene. Ähnliches gilt für die überschiessende Immunantwort bei Allergien.

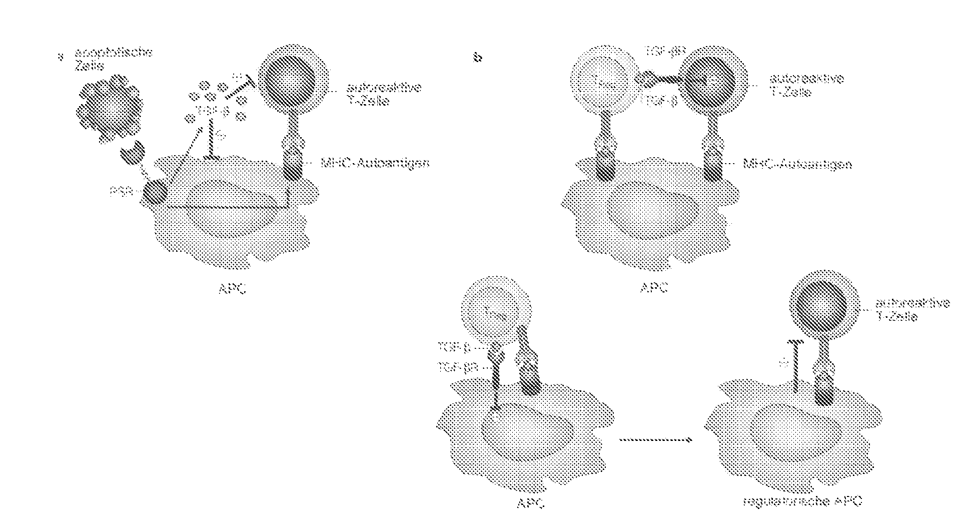

Abb. I: Inhibierung autoreaktiver T-Zellen über lösliches (a) oder membranständiges TGF-β1 (b). Membranständiges TGF-β1 auf Treg wirkt direkt inhibitorisch auf autoreaktive T-Zellen, indem es an deren TGF-β1-Rezeptor bindet (Zell-Zell-Kontakt) (b, oben). Dieser Zell-Kontakt kann über die gleichzeitige Bindung von sowohl Treg als auch autoreaktiver T-Zelle an eine antigenpräsentierende Zelle (APC, insbesondere dendritische Zellen), erreicht werden (oben). Andererseits können durch vorhergehende Bindung der Treg an die APC diese so verändert werden (Fehlen kostimulatorischer Signale), dass eine nachfolgend bindende autoreaktive T-Zelle nicht aktiviert wird (Anergie). In beiden Fällen zeichnen sich Treg und autoreaktive T-Zelle durch die gleiche Antigen-Spezifität aus. (Abbildung aus Nature Reviews in Immunology 2: 46-53, 2002.)

Erkenntnisse der letzten Jahre zeigen, dass im gesunden Organismus diese "Toleranz" aktiv aufrecht erhalten wird, indem autoreaktive T-Lymphozyten aktiv in ihrer Funktion und ihrem Wachstum unterdrückt werden. Dies wird durch eine spezielle, supprimierende T-Zell-Population, die sogenannten natürlichen regulatorischen T-Zellen (Treg, CD4⁺CD25⁺-Zellen), erreicht. Treg-Zellen entstehen im Thymus [Kawahata K. et al., J. Immunol. 168: 4399-4405, 2002] und machen im peripheren Blut einen Anteil von 5-10% der T-Zellen aus. Sie wirken auf CD4⁺-T-Zellen der gleichen Antigen-Spezifität über direkten Zell-Kontakt inhibitorisch. Diese inhibitorische Wirkung wird über eine starke Expression von TGF-β1 in/auf den Treg erreicht. Das TGF-β1 ist dabei auf der Oberfläche der Treg präsentiert und bindet an den TGF-β1-Rezeptor auf autoreaktiven T-Zellen, was einen völlig neuen Wirkungsmechanismus dieses starken immunsuppressiven Zytokins darstellt [Nakamura et al., J Exp Med 194: 629-644, 2001].

Treg-Zellen hemmen die Autoimmunität effizienter als die Immunantwort gegen "Fremd"-Antigene [Romagnoli P et al., J Immunol 168: 1644-1648, 2002]. Daher haben Einschränkungen oder Verluste der Funktion von Treg-Zellen besondere pathogenetische Bedeutung bei der Entstehung von Autoimmunerkrankungen. Ein direkter Zusammenhang zwischen Zahl/Funktion von Treg-Zellen und der Manifestation von Autoimmunerkrankungen ist für den Typ I-Diabetes [Boudaly S et al., Eur Cytokine Netw 13: 29-37, 2002; Gregori S et al., Diabetes 51: 1367-1374, 2002], die Autoimmun-Enzephalomyelitis (Tiermodell der Multiplen Sklerose) [Furtado GC et al., Immunol Rev 182: 122-134, 2001; Muhallab S et al., Scand J Immunol 55: 264-273, 2002; Hamilton NH et al., Scand J Immunol 55: 171-177, 2002], für die "autoimmune ovarian disease" (AOD) [Tung KS et al., Immunol Rev 182:135-148, 2001], sowie für den Morbus Crohn [Neurath MF et al., J Exp Med 195: 1129-1143, 2002] nachgewiesen worden.

Darüber hinaus sind Treg-Zellen auch für die Unterdrückung intestinaler oder pulmonaler Entzündungen verantwortlich [Singh B et al., Immunol Rev 182: 190-200, 2001; Hori S et al., Eur J Immunol 32: 1282-1291, 2002]. Ebenso eindeutig belegt ist die Rolle von Treg-Zellen für die Unterdrückung von Abstossungsepisoden nach allogener (Fremd-) Organtransplantation [Kingsley CI et al., J Immunol 168: 1080-1086, 2002; Taylor PA et al., Blood 99: 3493-3499, 2002; Chiffoleau E et al., J Immunol 168: 5058-5069, 2002]. All diesen immunsupprimierenden Funktionen der Treg-Zellen ist gemeinsam, dass sie sich durch eine hohe Antigenspezifität auszeichnen, d.h. jeder Treg-Zell-Klon ist gegen ein spezielles Antigen gerichtet und inhibiert autoreaktive T-Zellen der gleichen Antigenspezifität unter normalen physiologischen Bedingungen. Im Falle von Autoimmunerkrankungen geht diese Funktion der Treg verloren und autoreaktive T-Zell-Klone wie sie im Falle des Typ I Diabetes gegen Proteine der pankreatischen Beta-Zelle gerichtet sind, führen zum Ausbruch der Autoimmunerkrankung.

Diese Antigenspezifität kann auf der anderen Seite auch therapeutisch ausgenutzt werden, indem durch gezielte "antigen-spezifische" Aktivierung *in vivo* oder *ex vivo* von Treg- Zellen (bzw. von diese Zellen aktivierenden denritischen Zellen) die Zahl/Funktion dieser Zellen erhöht bzw. wieder hergestellt werden. Dazu ist auch die orale Applikation von "Antigenen" geeignet [Zhang et al., J Immunol 167: 4245-4253, 2001]. Die Herstellung solcher Antigene ist jedoch technisch äußerst zeit- und kostenintensiv und auf Antigen-spezifische T-Zell-Klone beschränkt.

Die besondere Rolle von TGF-β1 für die Regulation der immunologischen Hyperreaktivität wird durch zwei jüngere Publikationen unterstrichen, die zeigen, dass die durch genetische Manipulation bewirkte Überproduktion von TGF-β1 in CD4⁺-Zellen das Krankheitsgeschehen zu unterdrücken vermag. Da im Falle des Asthma Th2-Zellen an der Pathogenese entscheidend beteiligt sind, kann durch transgene Überproduktion von TGF-β1 demzufolge die Funktion pathogener Th2-Zell-Klone wirksam inhibiert werden [Hansen G et al., J Clin Invest 105: 61-70, 2000; Thorbecke GJ et al., Cytokine Growth Factor Rev 11: 89-96, 2000]. Der Nachteil dieser Verfahren zur Induktion der Produktion von TGF-β1 in CD4⁺- bzw. Treg-Zellen ist, daß sie eine genetische Manipulation erforderlich machen, die zum einen sehr aufwendig und zum anderen für eine pharmakologische Anwendung am Menschen oder Tier ungeeignet ist.

Der vorliegenden Erfindung liegt die Aufgabe zugrunde, eine effiziente Methode zur Induktion von Produktion und Expression von TGF-β1 in und/oder auf Treg-Zellen bereitzustellen, die darüber hinaus zur Vorbeugung und/oder Behandlung von Autoimmunerkrankungen, Allergien, Arteriosklerose und zur Unterdrückung von Transplantat-Abstossungen am Mensch oder Tier geeignet ist. Eine weitere Aufgabe ist es, entsprechende pharmazeutische Zubereitungen bereitzustellen, mit denen diese Aufgaben gelöst werden können.

Überraschenderweise wurde gefunden, daß Inhibitoren von Alanyl-Aminopeptidasen und/oder von Enzymen gleicher Substratspezifität die Produktion von TGF-β1 und Expression von TGF-β11 in und/oder auf Treg-Zellen induzieren und so zur Vorbeugung und Behandlung von Autoimmunerkrankungen, Allergien und Arteriosklerose geeignet sind und zur Unterdrückung von Transplantat-Abstossungen dienen können.

Die Erfindung betrifft daher die Verwendung von einem Inhibitor oder mehreren Inhibitoren von Alanyl-Aminopeptidasen und/oder von Enzymen gleicher Substratspezifität zur Induktion von Produktion von TGF-β1 und Expression von TGF-β1 in und/oder auf Treg-Zellen.

Der Zusammenhang zwischen der Inhibierung von Alanyl-Aminopeptidasen und/oder der Inhibierung von Enzymen gleicher Substratspezifität und der Induzierung der Produktion und Oberflächenexpression von TGF-β1 in und/oder auf Treg-Zellen ist bislang nicht bekannt gewesen.

Als Inhibitoren kommen alle Inhibitoren der Alanyl-Aminopeptidasen und alle Inhibitoren von Enzymen gleicher Substratspezifität in Betracht. Bevorzugt kommen Actinonin, Leuhistin, Phebestin, Amastatin, Bestatin, Probestin, Arphamenin, MR 387, β-Aminothiole, α-Aminophosphinsäuren und deren Ester und Salze, α -Aminophosphonate, α-Aminoboronsäuren, α-Aminoaldehyde, Hydroxamate von α-Aminosäuren, N-Phenylphthalimide, N-Phenylhomophthalimide, α-Ketoamide, Thalidomid und dessen Derivate zum Einsatz. Besonders bevorzugt unter diesen sind α-Ketoamide, α-Aminophosphinsäuren, N-Phenylhomophthalimide, α-Aminophosphonate und Phebestin, wobei besonders bevorzugt als α-Ketoamide 3-Amino-2-oxo-4-phenylbutansäureamide, als α-Aminophosphinsäuren D-Phe-γ[PO(OH)-CH₂]-Phe-Phe, als N-Phenylhomophthalimide PAQ-22, als α-Aminophosphonate RB3014 und/oder Phebestin, und ganz besonders bevorzugt PAQ-22, RB3014 und/oder Phebestin eingesetzt werden.

Vorteilhaft an den bevorzugten und den besonders bevorzugten Inhibitoren ist insbesondere die einfache Zugänglichkeit, der geringe Preis und die einfache galenische Verarbeitbarkeit.

Als Enzym mit gleicher Substratspezifität wie die Alanyl-Aminopeptidasen sei hier beispielhaft die zytosolische Alanyl-Aminopeptidase genannt. Für die zytosolische Alanyl-Aminopeptidase ist PAQ-22 spezifisch. Als bevorzugter Inhibitor der zytosolischen Alanyl-Aminopeptidase wird daher PAQ-22 verwendet, oder eine Mischung mehrerer Inhibitoren eingesetzt, die PAQ-22 umfaßt.

Die Hemmung der enzymatischen Aktivität der membranständigen Alanyl-Aminopeptidase (APN, CD13, EC 3.4.11.2) oder die Hemmung von Enzymen mit gleicher Substratspezifität und Inhibitorsensitivität (wie z.B. der zytosolischen Alanyl-Aminopeptidase, zAAP, PSA, EC3.4.11.14) erhöht die Genexpression von TGF-β1 in Treg-Zellen sowie die Expression des immunsuppressiven Zytokins TGF-β1 ("transforming growth factor β1") in/auf regulatorischen Zellen. Diese Induktion der Produktion und insbesondere der Oberflächenexpression von TGF-β1 bewirkt selektiv die Stärkung bzw. Restitution der Funktion von Treg-Zellen und ist aufgrund der oben genannten Zusammenhänge zwischen der Expression von TGF-β1 auf den Treg-Zellen und der inhibitorischen Wirkung auf autoreaktive T-Lymphozyten geeignet, die bei Autoimmunerkrankungen und entzündlichen Erkrankungen, bei Allergien sowie bei Abstossungsepisoden nach Organtransplantation bestehenden funktionellen Defizite der Treg-Zellen zu überwinden und somit die Vorbeugung dieser Erkrankungen zu erlauben und/oder den Verlauf bzw. die Schwere dieser Erkrankungen zu verbessern und/oder diese Erkrankungen zu heilen. Alle diese Erkrankungen und die Abstossungsepisoden nach Organtransplantation sind durch das Fehlen eines ausreichend wirksamen natürlichen immunsuppressiven Prinzips, d. h. nicht ausreichend funktionierende immunregulatorische Zellen einschließlich einer defizienten Produktion von TGF-β1 gekennzeichnet. Die erfindungsgemäße Induktion von TGF-β1 ist nicht auf einzelne Antigen-spezifische T-Zell-Klone beschränkt.

Daher betrifft die Erfindung auch die Verwendung von einem Inhibitor oder mehreren Inhibitoren von Alanyl-Aminopeptidasen und/oder von Enzymen gleicher Substratspezifität zur Vorbeugung und/oder Behandlung von Autoimmunerkrankungen. Erfindungsgemäß bevorzugt ist die Verwendung zur Vorbeugung und/oder Behandlung von Rheumatoider Arthritis, Lupus erythematodes, Multipler Sklerose, IDDM, Morbus Crohn, Colitis Ulcerosa, Psoriasis, Neurodermitis, Glomerulonephritis, interstitieller Nephritis, Vaskulitis, autoimmuner Schilddrüsenerkrankungen, autoimmunhämolytischer Anämie, oder anderen chronischen Erkrankungen mit entzündlicher Genese wie beispielsweise der Arteriosklerose. Besonders bevorzugt ist die Verwendung zur Vorbeugung und/oder Behandlung von Multipler Sklerose oder Arteriosklerose.

Erfindungsgemäß werden ein Inhibitor oder mehrere Inhibitoren von Alanyl-Aminopeptidasen und/oder von Enzymen gleicher Substratspezifität auch zur Vorbeugung und/oder Behandlung von Allergien vom Typ I nach Gell und Coombs oder Allergien vom Typ II, III oder IV eingesetzt. Dabei ist die Verwendung zur Vorbeugung und/oder Behandlung von Asthma bronchiale oder Heuschnupfen als Allergie vom Typ I nach Gell und Coombs und/oder Kontaktallergien als Allergien vom Typ II, III oder IV bevorzugt.

Die Erfindung betrifft weiter die Verwendung von einem Inhibitor oder mehreren Inhibitoren von Alanyl-Aminopeptidasen und/oder von Enzymen gleicher Substratspezifität zur Unterdrückung von Transplantat-Abstossungen, bevorzugt bei Nieren- oder Knochenmarktransplantationen.

Bei der Vorbeugung und/oder Behandlung von Autoimmunerkrankungen, Allergien und Heuschnupfen und bei der Unterdrückung von Transplantat-Abstossungen kommen als Inhibitoren alle Inhibitoren der Alanyl-Aminopeptidasen und alle Inhibitoren von Enzymen gleicher Substratspezifität in Betracht. Bevorzugt werden Actinonin, Leuhistin, Phebestin, Amastatin, Bestatin, Probestin, Arphamenin, MR 387, β-Aminothiole, α-Aminophosphinsäuren und deren Ester und Salze, α -Aminophosphonate, α-Aminoboronsäuren, α-Aminoaldehyde, Hydroxamate von α-Aminosäuren, N-Phenylphthalimide, N-Phenylhomophthalimide, α-Ketoamide, Thalidomid und dessen Derivate eingesetzt. Besonders bevorzugt unter diesen sind α-Ketoamide, α-Aminophosphinsäuren, N-Phenylhomophthalimide, α-Aminophosphonate und Phebestin, wobei besonders bevorzugt als α-Ketoamide 3-Amino-2-oxo-4-phenylbutansäureamide, als α-Aminophosphinsäuren D-Phe-γ[PO(OH)-CH₂]-Phe-Phe, als N-Phenylhomophthalimide PAQ-22, als α-Aminophosphonate RB3014 und/oder Phebestin, und ganz besonders bevorzugt PAQ-22, RB3014 und/oder Phebestin eingesetzt werden.

Als Enzym mit gleicher Substratspezifität wie die Alanyl-Aminopeptidasen sei hier beispielhaft die zytosolische Alanyl-Aminopeptidase genannt. Für die zytosolische Alanyl-Aminopeptidase ist PAQ-22 spezifisch. Als bevorzugter Inhibitor der zytosolischen Alanyl-Aminopeptidase wird daher PAQ-22 verwendet, oder eine Mischung mehrerer Inhibitoren eingesetzt, die PAQ-22 umfaßt.

Wegen der hohen Antigenspezifität der Treg-Zell-Klone scheint die gemeinsame Applikation von Aminopeptidase-Inhibitoren mit den die jeweilige Erkrankung verursachenden Antigenen, zum Beispiel antigene Peptide des "myelin basic proteins" bei der Multiplen Sklerose, für die Therapie besonders geeignet, da auf diese Weise gezielt und spezifisch die Hemmung der pathogenen T-Zell-Klone bewirkt wird. Die Antigen-spezifische Immunsuppression ist praktisch nebenwirkungsfrei [Zhang et al., J Immunol 167: 4245-4253, 2001].

Daher betrifft die Erfindung auch die Verwendung von Inhibitoren von Alanyl-Aminopeptidasen und/oder von Enzymen gleicher Substratspezifität zur Induktion von Produktion von TGF-β1 und Expression von TGF-β1 in und/oder auf Treg-Zellen und zur Vorbeugung und/oder Behandlung von Autoimmunerkrankungen, Allergien, Heuschnupfen, Arteriosklerose sowie zur Unterdrückung von Transplantat-Abstossungen, bei der zusätzlich Peptidfragmente von pathogenen Autoantigenen oder synthetische Analoga und/oder spezifische antigene Komponenten pathogener Mikroorganismen eingesetzt werden. Die oben aufgeführten in Frage kommenden Inhibitoren und die bevorzugten und besonders bevorzugten Inhibitoren sowie die bevorzugten und besonders bevorzugten Erkrankungen sind auch bei der Verwendung bevorzugt, bei der zusätzlich Peptidfragmente von pathogenen Autoantigenen oder synthetische Analoga und/oder spezifische antigene Komponenten pathogener Mikroorganismen eingesetzt werden. Bevorzugte Peptidfragmente von pathogenen Autoantigenen bei der Multiplen Sklerose sind MBP (Myelin-Basisches-Protein), MOG (Myelin-Oligodendrozyten-Glykoprotein), MAG (Myelin-Assoziiertes-Glykoprotein) und PLP (Proteolipid-Protein), bevorzugte spezifische antigene Komponenten pathogener Mikroorganismen sind Hüllproteine oder Membranglycolipid-Komplexe.

Vorteilhaft an den bevorzugten und den besonders bevorzugten Inhibitoren ist insbesondere die einfache Zugänglichkeit, der geringe Preis und die einfache galenische Verarbeitbarkeit.

Daher betrifft die Erfmdung auch die Verwendung von einem Inhibitor oder mehreren Inhibitoren von Alanyl-Aminopeptidasen und/oder von Enzymen gleicher Substratspezifität zur Herstellung eines Arzneimittels oder einer pharmazeutischen Zubereitung zur Induktion von Produktion von TGF-β1 und Expression von TGF-β1 in und/oder auf Treg-Zellen.

Als Inhibitoren kommen alle Inhibitoren der Alanyl-Aminopeptidasen und alle Inhibitoren von Enzymen gleicher Substratspezifität in Betracht. Bevorzugt kommen Actinonin, Leuhistin, Phebestin, Amastatin, Bestatin, Probestin, Arphamenin, MR 387, β-Aminothiole, α-Aminophosphinsäuren und deren Ester und Salze, α -Aminophosphonate, α-Aminoboronsäuren, α-Aminoaldehyde, Hydroxamate von α-Aminosäuren, N-Phenylphthalimide, N-Phenylhomophthalimide, α-Ketoamide, Thalidomid und dessen Derivate zum Einsatz. Besonders bevorzugt unter diesen sind α-Ketoamide, α-Aminophosphinsäuren, N-Phenylhomophthalimide, α-Aminophosphonate und Phebestin, wobei besonders bevorzugt als α-Ketoamide 3-Amino-2-oxo-4-phenylbutansäureamide, als α-Aminophosphinsäuren D-Phe-γ[PO(OH)-CH₂]-Phe-Phe, als N-Phenylhomophthalimide PAQ-22, als α-Aminophosphonate RB3014 und/oder Phebestin, und ganz besonders bevorzugt PAQ-22, RB3014 und/oder Phebestin eingesetzt werden.

Als Enzym mit gleicher Substratspezifität wie die Alanyl-Aminopeptidasen sei hier beispielhaft die zytosolische Alanyl-Aminopeptidase genannt. Für die zytosolische Alanyl-Aminopeptidase ist PAQ-22 spezifisch. Als bevorzugter Inhibitor der zytosolischen Alanyl-Aminopeptidase wird daher PAQ-22 verwendet, oder eine Mischung mehrerer Inhibitoren eingesetzt, die PAQ-22 umfaßt.

Die Erfmdung betrifft auch die Verwendung von einem Inhibitor oder mehreren Inhibitoren von Alanyl-Aminopeptidasen und/oder von Enzymen gleicher Substratspezifität zur Herstellung eines Arzneimittels oder einer pharmazeutischen Zubereitung zur Vorbeugung und/oder Behandlung von Autoimmunerkrankungen, von Allergien vom Typ I nach Gell und Coombs, wie Heuschnupfen, von Allergien vom Typ II, III oder IV und die Verwendung zur Herstellung eines Arzneimittels oder einer pharmazeutischen Zubereitung zur Unterdrückung von Transplantat-Abstossungen. Bevorzugte Erkrankungen und Transplantationsarten sind in den Unteransprüchen 26, 27, 29 und 31 aufgeführt. Bevorzugte Inhibitoren von Alanyl-Aminopeptidasen und Enzyme gleicher Substratspezifität sind in den Ansprüchen 32 bis 35 genannt.

Bei der Verwendung von einem Inhibitor oder mehreren Inhibitoren von Alanyl-Aminopeptidasen und/oder von Enzymen gleicher Substratspezifität zur Herstellung eines Arzneimittels oder einer pharmazeutischen Zubereitung zur Vorbeugung und/oder Behandlung von Autoimmunerkrankungen, von Allergien vom Typ I nach Gell und Coombs, wie Heuschnupfen, von Allergien vom Typ II, III oder IV und bei der Verwendung zur Herstellung eines Arzneimittels oder einer pharmazeutischen Zubereitung zur Unterdrückung von Transplantat-Abstossungen können zusätzlich Peptidfragmente von pathogenen Autoantigenen bei Multipler Sklerose oder synthetische Analoga und/oder spezifische antigene Komponenten pathogener Mikroorganismen eingesetzt werden, wobei als Peptidfragmente von pathogenen Autoantigenen bevorzugt MBP (Myelin-Basisches-Protein), MOG (Myelin-Oligodendrozyten-Glykoprotein), MAG (Myelin-Assoziiertes-Glykoprotein) und/oder PLP (Proteolipid-Protein) und als spezifische antigene Komponenten pathogener Mikroorganismen Hüllproteine oder Membranglycolipid-Komplexe eingesetzt werden.

Des weiteren betrifft die vorliegende Erfindung eine pharmazeutische Zubereitung, umfassend einen Inhibitor oder mehrere Inhibitoren von Alanyl-Aminopeptidasen und/oder Enzyme gleicher Substratspezifität sowie einen oder mehrere pharmakologisch unbedenkliche Träger-, Zusatz- und/oder Hilfsstoffe. Die Erfindung betrifft ebenso eine pharmazeutische Zubereitung, die einen Inhibitor oder mehrere Inhibitoren von Alanyl-Aminopeptidasen und/oder Enzyme gleicher Substratspezifität und Peptidfragmente von pathogenen Autoantigenen oder synthetische Analoga und/oder spezifische antigene Komponenten pathogener Mikroorganismen sowie einen oder mehrere pharmakologisch unbedenkliche Träger-, Zusatz-und/oder Hilfsstoffe umfaßt.

Die Erfmdung zeigt, dass zur Therapie von entzündlichen Erkrankungen und AutoimmunErkrankungen sowie allogenen Abstossungsreaktionen und Allergien, für deren Entstehung die Proliferation und die Aktivierung von pathogenen T-Zell-Klonen eine zentrale Rolle Bedeutung hat, die Applikation von Hemmstoffen der oben genannten Enzyme bzw. entsprechender Zubereitungen und Darreichungsformen daraus geeignet sind. Die gleichzeitige Applikation von krankheitsspezifischen Antigenen erhöht diesen therapeutischen Effekt weiter und engt die Wirkung auf den pathogenetisch relevanten Prozeß ein.

Die Applikation von Aminopeptidase-Inhibitoren zur Induktion der TGF-β1- Expression in/auf Treg-Zellen und damit zur Erhöhung der immunsuppressiven Funktion dieser pathogenetisch bedeutsamen inhibitorischen T-Zell-Population stellt bei den genannten Erkrankungen eine neuartige Methode und ergänzende, in den zentralen pathogenetischen Vorgang eingreifende Therapieform dar.

Der Inhibitor oder die mehreren Inhibitoren von Alanyl-Aminopeptidasen und/oder von Enzymen gleicher Substratspezifität können in pharmazeutisch anwendbaren Formulierungskomplexen als Inhibitoren, Substrate, Pseudosubstrate, inhibitorisch wirkende Peptide und Peptidderivate sowie als Antikörper dieses Enzyms zur Anwendung kommen. Bevorzugte Inhibitoren sind Bestatin, Phebestin, Probestin, Actinonin, Leuhistin, RB3014, PAQ-22 und deren Derivate, besonders bevorzugt sind Phebestin, RB3014 und/oder PAQ-22.

Die Verabreichung kann in allen geeigneten Formen geschehen, so zum Beispiel als topische Applikation in Form von Cremes, Salben, Pasten, Gelen, Lösungen, Sprays, Liposomen, Schüttelmixturen, Hydrokolloidverbänden bzw. anderen dermatologischen Grundlagen/Vehikeln einschließlich instillativer Applikation oder als systemische Applikation zur oralen, transdermalen, intravenösen, subcutanen, intracutanen, inhalativen, intramuskulären Anwendung in geeigneten Rezepturen bzw. in geeigneter Galenik oder in Anbindung an oder Einarbeitung in Mikropartikel zu Überwindung der Blut-Hirn-Schranke. Gleichzeitig kann die Applikation von "krankheitsspezifischen" Antigenen (Petidfragmente, Lipopolysaccharide u.a.) in entsprechenden Darreichungsformen den Therapieerfolg steigern.

Die Erfmdung wird unter Bezugnahme auf die nachfolgenden Beispiele näher erläutert, ohne auf diese beschränkt zu sein.

### Beispiel 1

### Induktion der Oberflächenexpression von TGF-β1 auf humanen regulatorischen T-Lymphozyten (CD4⁺CD25⁺) nach Inkubation mit dem Inhibitor Phebestin.

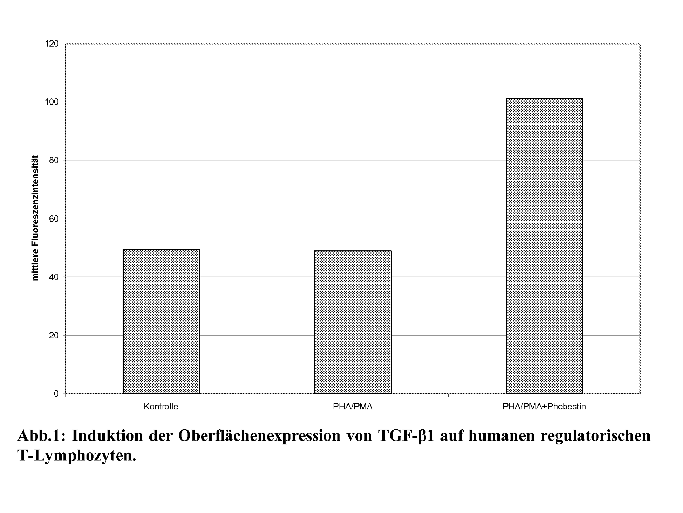

Die T-Zellen wurden 24h ohne Zusatz (Kontrolle), unter Zugabe von PHA und PMA und unter gleichzeitiger Zugabe von PHA/PMA mit Phebestin inkubiert. PHA (Phythaemagglutinin) und PMA (Phorbol-Myristat-Acetat) wurden als Mitogene und die Proliferation stimulierend Agentien eingesetzt. Anschließend erfolgte die Messung der Oberflächenexpression von TGF-β1 mittels Durchflußzytometrie unter Verwendung des kommerziell verfügbaren polyklonalen anti-TGF-β1 Antikörpers (Huhn-anti-Human; R & D Systems). Die Ergebnisse sind in Abbildung 1 dargestellt.

### Beispiel 2

### Induktion der Genexpression von TGF-β1 in humanen regulatorischen T-Lymphozyten (CD4⁺CD25⁺) nach Inkubation mit dem Inhibitor Phebestin.

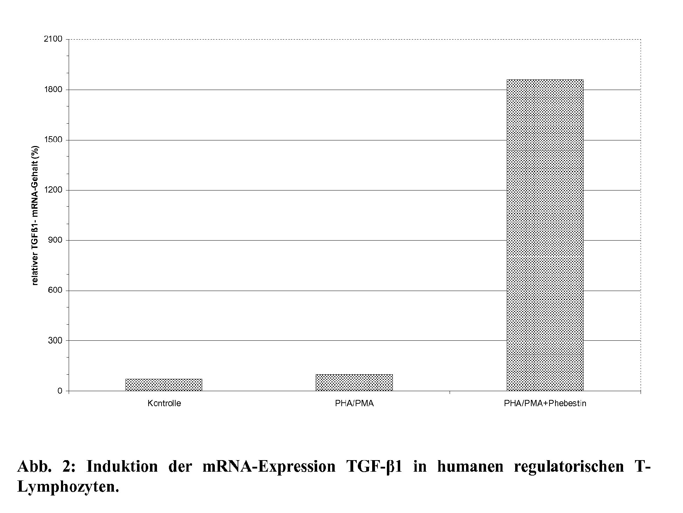

Die T-Zellen wurden 24h ohne Zusatz (Kontrolle), unter Zugabe von PHA und PMA oder unter gleichzeitiger Zugabe von PHA/PMA mit Phebestin inkubiert. PHA (Phythaemagglutinin) und PMA (Phorbol-Myristat-Acetat) wurden als Mitogene und die Proliferation stimulierend Agentien eingesetzt. Anschließend wurde mittels quantitativer RT-PCR unter Verwendung des i-Cyclers der Gehalt an TGFβ1-mRNA bestimmt. Die Ergebnisse sind in Abbildung 2 dargestellt.

### Beispiel 3

### Induktion der Genexpression von TGF-β1 auf humanen regulatorischen T-Lymphozyten (CD4⁺CD25⁺) nach Inkubation mit dem Inhibitor PAQ-22.

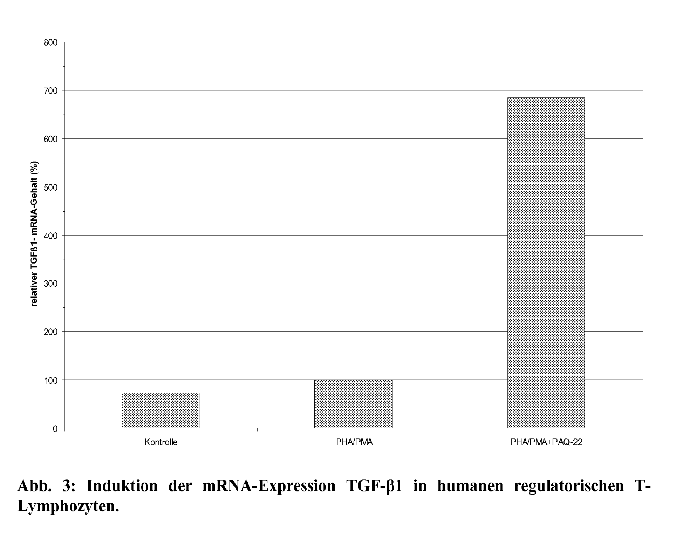

Die T-Zellen wurden 24h ohne Zusatz (Kontrolle), unter Zugabe von PHA und PMA oder unter gleichzeitiger Zugabe von PHA/PMA mit PAQ-22 inkubiert. PHA (Phythaemagglutinin) und PMA (Phorbol-Myristat-Acetat) wurden als Mitogene und die Proliferation stimulierend Agentien eingesetzt. Anschließend wurde mittels quantitativer RT-PCR unter Verwendung des i-Cyclers der Gehalt an TGFβ1-mRNA bestimmt. Die Ergebnisse sind in Abbildung 3 dargestellt.

### Beispiel 4

### Induktion der Genexpression von TGF-β1 auf humanen regulatorischen T-Lymphozyten (CD4⁺CD25⁺) nach Inkubation mit dem Aminopeptidase-Inhibitor RB3014.

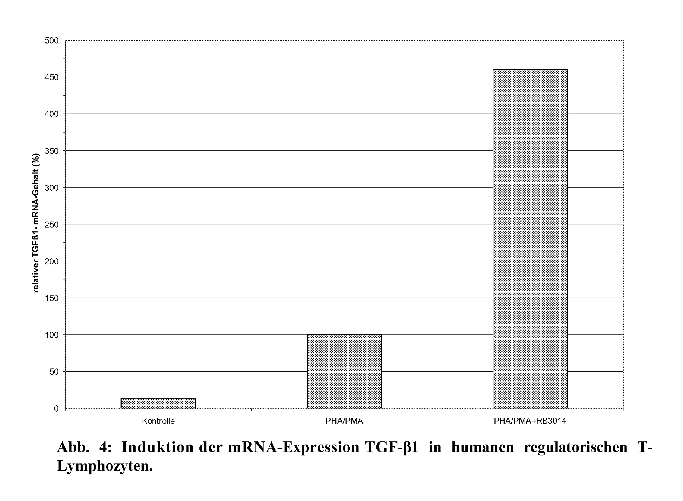

Die T-Zellen wurden 72h ohne Zusatz (Kontrolle), unter Zugabe von PHA und PMA oder unter gleichzeitiger Zugabe von PHA/PMA mit RB3014 inkubiert. PHA (Phythaemagglutinin) und PMA (Phorbol-Myristat-Acetat) wurden als Mitogene und die Proliferation stimulierend Agentien eingesetzt. Anschließend wurde mittels quantitativer RT-PCR unter Verwendung des i-Cyclers der Gehalt an TGF-β1-mRNA bestimmt. Die Ergebnisse sind in Abbildung 4 dargestellt.

Die vorliegende Erfindung betrifft die Verwendung von einem Inhibitor oder mehreren Inhibitoren von Alanyl-Aminopeptidasen und/oder von Enzymen gleicher Substratspezifität zur Induktion von Produktion von TGF-β1 und Expression von TGF-β1 in und/oder auf Treg-Zellen.

Insbesondere betrifft die vorliegende Erfindung die Verwendung von einem Inhibitor oder mehreren Inhibitoren der Alanyl-Aminopeptidasen und/oder von Enzymen gleicher Substratspezifität, die ausgewählt sind aus der Gruppe, die besteht aus Actinonin, Leuhistin, Phebestin, Amastatin, Bestatin, Probestin, Arphamenin, MR 387, β-Aminothiole, α-Aminophosphinsäuren und deren Ester und Salze, α -Aminophosphonate, α-Aminoboronsäuren, α-Aminoaldehyde, Hydroxamate von α-Aminosäuren, N-Phenylphthalimide, N-Phenyl-homophthalimide, α-Ketoamide, Thalidomid und dessen Derivate ist.

Bevorzugt werden als der eine Inhibitor oder die mehreren Inhibitoren α-Ketoamide, bevorzugt 3-Amino-2-oxo-4-phenylbutansäureamide, α-Aminophosphinsäuren, bevorzugt D-Phe-γ[PO(OH)-CH₂]-Phe-Phe, N-Phenylhomophthalimide, bevorzugt PAQ-22, α-Aminophosphonate, bevorzugt RB3014 und/oder Phebestin, besonders bevorzugt PAQ-22, RB3014 und/oder Phebestin, eingesetzt.

Vorzugsweise ist das Enzym gleicher Substratspezifität zytosolische Alanyl-Aminopeptidase. Im Falle der zytosolischen Alanyl-Aminopeptidase wird bevorzugt als der eine Inhibitor PAQ-22 eingesetzt oder umfassen die mehreren Inhibitoren PAQ-22.

Insbesondere betrifft die vorliegende Erfindung die Verwendung von einem Inhibitor oder mehreren Inhibitoren von Alanyl-Aminopeptidasen und/oder von Enzymen gleicher Substratspezifität zur Vorbeugung und/oder Behandlung von Autoimmunerkrankungen wie Rheumatoider Arthritis, Lupus erythematodes, Multipler Sklerose, IDDM, Morbus Crohn, Colitis Ulcerosa, Psoriasis, Neurodermitis, Glomerulonephritis, interstitieller Nephritis, Vaskulitis, autoimmuner Schilddrüsenerkrankungen, autoimmunhämolytischer Anämie, oder anderen chronischen Erkrankungen mit entzündlicher Genese wie Arteriosklerose, vorzugsweise zur Vorbeugung und/oder Behandlung von Multipler Sklerose oder Arteriosklerose.

In einer Alternative betrifft die vorliegende Erfindung die Verwendung von einem Inhibitor oder mehreren Inhibitoren von Alanyl-Aminopeptidasen und/oder von Enzymen gleicher Substratspezifität zur Vorbeugung und/oder Behandlung von Allergien vom Typ I nach Gell und Coombs, Heuschnupfen oder Allergien vom Typ II, III oder IV, insbesondere von Asthma bronchiale oder Heuschnupfen als Allergie vom Typ I nach Gell und Coombs und/oder Kontaktallergien als Allergien vom Typ II, III oder IV.

In einer weiteren Alternative betrifft die vorliegende Erfindung die Verwendung von einem Inhibitor oder mehreren Inhibitoren von Alanyl-Aminopeptidasen und/oder von Enzymen gleicher Substratspezifität zur Unterdrückung von Transplantat-Abstossungen, insbesondere bei Nieren- oder Knochenmarktransplantationen.

In einer bevorzugten Ausführungsform der vorliegenden Erfindung werden zusätzlich Peptidfragmente von pathogenen Autoantigenen oder synthetische Analoga und/oder spezifische antigene Komponenten pathogener Mikroorganismen eingesetzt. Besonders bevorzugt werden als Peptidfragmente von pathogenen Autoantigenen MBP (Myelin-Basisches-Protein), MOG (Myelin-Oligodendrozyten-Glykoprotein), MAG (Myelin-Assoziiertes-Glykoprotein) und/oder PLP (Proteolipid-Protein) eingesetzt und als spezifische antigene Komponenten pathogener Mikroorganismen Hüllproteine oder Membranglycolipid-Komplexe eingesetzt.

In einem weiteren Aspekt betrifft die vorliegend Erfindung die Verwendung von einem Inhibitor oder mehreren Inhibitoren von Alanyl-Aminopeptidasen und/oder von Enzymen gleicher Substratspezifität zur Herstellung eines Arzneimittels oder einer pharmazeutischen Zubereitung zur Induktion von Produktion von TGF-β1 und Expression von TGF-β1 in und/oder auf Treg-Zellen.

Bei der erfindungsgemäßen Verwendung ist der eine Inhibitor oder sind die mehreren Inhibitoren der Alanyl-Aminopeptidasen und/oder von Enzymen gleicher Substratspezifität mindestens ein Mitglied, gewählt aus der Gruppe, bestehend aus Actinonin, Leuhistin, Phebestin, Amastatin, Bestatin, Probestin, Arphamenin, MR 387, β-Aminothiole, α-Aminophosphinsäuren und deren Ester und Salze, α -Aminophosphonate, α-Aminoboronsäuren, α-Aminoaldehyde, Hydroxamate von α-Aminosäuren, N-Phenylphthalimide, N-Phenyl-homophthalimide, α-Ketoamide, Thalidomid und dessen Derivate.

Besonders bevorzugt werden als der eine Inhibitor oder die mehreren Inhibitoren α-Ketoamide, bevorzugt 3-Amino-2-oxo-4-phenylbutansäureamide, α-Aminophosphinsäuren, bevorzugt D-Phe-γ[PO(OH)-CH₂]-Phe-Phe, N-Phenylhomophthalimide, bevorzugt PAQ-22, α -Aminophosphonate, bevorzugt RB3014 und/oder Phebestin, besonders bevorzugt PAQ-22, RB3014 und/oder Phebestin, eingesetzt.

Bevorzugt verwendet werden der Inhibitor oder die mehreren Inhibitoren von Alanyl-Aminopeptidasen und/oder von Enzymen gleicher Substratspezifität zur Herstellung eines Arzneimittels oder einer pharmazeutischen Zubereitung zur Vorbeugung und/oder Behandlung von Autoimmunerkrankungen, insbesondere zur Vorbeugung und/oder Behandlung von Rheumatoider Arthritis, Lupus erythematodes, Multipler Sklerose, IDDM, Morbus Crohn, Colitis Ulcerosa, Psoriasis, Neurodermitis, Glomerulonephritis, interstitieller Nephritis, Vaskulitis, autoimmuner Schilddrüsenerkrankungen, autoimmunhämolytischer Anämie, oder anderen chronischen Erkrankungen mit entzündlicher Genese wie Arteriosklerose.

Darüber hinaus werden der eine Inhibitor oder die mehreren Inhibitoren von Alanyl-Aminopeptidasen und/oder von Enzymen gleicher Substratspezifität verwendet zur Herstellung eines Arzneimittels oder einer pharmazeutischen Zubereitung zur Vorbeugung und/oder Behandlung von Allergien vom Typ I nach Gell und Coombs, Heuschnupfen oder Allergien vom Typ II, III oder IV, insbesondere zur Vorbeugung und/oder Behandlung von Asthma bronchiale oder Heuschnupfen als Allergie vom Typ I nach Gell und Coombs und/oder Kontaktallergien als Allergien vom Typ II, III oder IV.

In einer weiteren Alternative werden der eine Inhibitor oder die mehreren Inhibitoren von Alanyl-Aminopeptidasen und/oder von Enzymen gleicher Substratspezifität verwendet zur Herstellung eines Arzneimittels oder einer pharmazeutischen Zubereitung zur Unterdrückung von Transplantat-Abstossungen, insbesondere bei Nieren- oder Knochenmarktransplantationen.

Vorzugsweise ist der eine Inhibitor oder die mehreren Inhibitoren der Alanyl-Aminopeptidasen und/oder von Enzymen gleicher Substratspezifität mindestens ein Mitglied gewählt aus der Gruppe, bestehend aus Actinonin, Leuhistin, Phebestin, Amastatin, Bestatin, Probestin, Arphamenin, MR 387, β-Aminothiole, α-Aminophosphinsäuren und deren Ester und Salze, α - Aminophosphonate, α-Aminoboronsäuren, α-Aminoaldehyde, Hydroxamate von α-Aminosäuren, N-Phenylphthalimide, N-Phenylhomophthalimide, α-Ketoamide, Thalidomid und dessen Derivate.

Bevorzugt werden als der eine Inhibitor oder die mehreren Inhibitoren α-Ketoamide, bevorzugt 3-Amino-2-oxo-4-phenylbutansäureamide, α-Aminophosphinsäuren, bevorzugt D-Phe-γ[PO(OH)-CH₂]-Phe-Phe, N-Phenylhomophthalimide, bevorzugt PAQ-22, α-Aminophosphonate, bevorzugt RB3014 und/oder Phebestin, besonders bevorzugt PAQ-22, RB3014 und/oder Phebestin eingesetzt.

Noch weiter bevorzugt werden der eine Hinhibitor oder die mehreren Inhibitoren der Alanyl-Aminopeptidasen und/oder von Enzymen gleicher Substratspezifität in Kombination mit Peptidfragmenten von pathogenen Autoantigenen oder synthetischen Analoga und/oder spezifischen antigenen Komponenten pathogener Mikroorganismen eingesetzt.

Vorzugsweise werden als Peptidfragmente von pathogenen Autoantigenen MBP (Myelin-Basisches-Protein), MOG (Myelin-Oligodendrozyten-Glykoprotein), MAG (Myelin-Assoziiertes-Glykoprotein) und/oder PLP (Proteolipid-Protein) und als spezifische antigene Komponenten pathogener Mikroorganismen Hüllproteine oder Membranglycolipid-Komplexe eingesetzt.

Darüber hinaus betrifft die vorliegende Erfindung eine pharmazeutische Zubereitung, umfassend einen Inhibitor oder mehrere Inhibitoren von Alanyl-Aminopeptidasen und/oder Enzyme gleicher Substratspezifität sowie einen oder mehrere pharmakologisch unbedenkliche Träger-, Zusatz- und/oder Hilfsstoffe.

In einer bevorzugten Ausführungsform umfaßt die pharmazeutische Zubereitung einen Inhibitor oder mehrere Inhibitoren von Alanyl-Aminopeptidasen und/oder Enzyme gleicher Substratspezifität und Peptidfragmente von pathogenen Autoantigenen oder synthetische Analoga und/oder spezifische antigene Komponenten pathogener Mikroorganismen sowie einen oder mehrere pharmakologisch unbedenkliche Träger-, Zusatz- und/oder Hilfsstoffe.

## Patentansprüche

1. Verwendung von einem Inhibitor oder mehreren Inhibitoren von Alanyl-Aminopeptidasen und/oder von Enzymen gleicher Substratspezifität in Kombination mit Peptidfragmenten von pathogenen Autoantigenen oder synthetischen Analoga und/oder spezifischen antigenen Komponenten pathogener Mikroorganismen zur Herstellung einer pharmazeutischen Zubereitung zur Vorbeugung und/oder Behandlung von Autoimmunerkrankungen, zur Vorbeugung und/oder Behandlung von Allergien vom Typ I nach Gell und Coombs oder Allergien vom Typ II, III oder IV und/oder zur Unterdrückung von Transplantat-Abstoßungen.

2. Verwendung nach Anspruch 1, bei der der eine Inhibitor oder die mehreren Inhibitoren der Alanyl-Aminopeptidasen und/oder von Enzymen gleicher Substratspezifität mindestens ein Mitglied ist/sind, gewählt aus der Gruppe, bestehend aus Actinonin, Leuhistin, Phebestin, Amastatin, Bestatin, Probestin, Arphamenin, MR 387, β-Aminothiole, α-Aminophosphinsäuren und deren Ester und Salze, α-Aminophosphonate, α-Aminoboronsäuren, α-Aminoaldehyde, Hydroxamate von α-Aminosäuren, N-Phenylphthalimide, N-Phenylhomophthalimide, α-Ketoamide, Thalidomid und dessen Derivate.

3. Verwendung nach Anspruch 2, bei der als der eine Inhibitor oder die mehreren Inhibitoren der Alanyl-Aminopeptidase und/oder von Enzymen gleicher Substratspezifität α-Ketoamide, 3-Amino-2-oxo-4-phenylbutansäureamide, α-Aminophosphinsäuren, D-Phe-γ[PO(OH)-CH₂]-Phe-Phe, N-Phenylhomophthalimide, PAQ-22, α-Aminophosphonate, RB3014 und/oder Phebestin eingesetzt werden.

4. Verwendung nach Anspruch 3, bei der als der eine Inhibitor oder die mehreren Inhibitoren der Alanyl-Aminopeptidase und/oder von Enzymen gleicher Substratspezifität PAQ-22, RB3014 und/oder Phebestin eingesetzt werden.

5. Verwendung nach einem der Ansprüche 1 bis 4, bei der als Enzym gleicher Substratspezifität zytosolische Alanyl-Aminopeptidase dient.

6. Verwendung nach Anspruch 5, bei der als der eine Inhibitor PAQ-22 eingesetzt wird oder die mehreren Inhibitoren PAQ-22 umfassen.

7. Verwendung nach einem der Ansprüche 1 bis 6, bei der als Peptidfragmente von pathogenen Autoantigenen MBP (Myelin-Basisches-Protein), MOG (Myelin-Oligodendrozyten-Glykoprotein), MAG (Myelin-Assoziiertes-Glykoprotein) und/ oder PLP (Proteolipid-Protein) eingesetzt werden.

8. Verwendung nach einem der Ansprüche 1 bis 7, bei der als spezifische antigene Komponenten pathogener Mikroorganismen Hüllproteine oder Membranglycolipid-Komplexe eingesetzt werden.

9. Verwendung nach einem der Ansprüche 1 bis 8 zur Vorbeugung und/oder Behandlung von Rheumatoider Arthritis, Lupus erythematodes, Multipler Sklerose, IDDM, Morbus Crohn, Colitis Ulcerosa, Psoriasis, Neurodermitis, Glomerulonephritis, interstitieller Nephritis, Vaskulitis, autoimmuner Schilddrüsenerkrankungen, autoimmunhämolytischer Anämie, oder anderen chronischen Erkrankungen mit entzündlicher Genese wie Arteriosklerose.

10. Verwendung nach einem der Ansprüche 1 bis 8 zur Vorbeugung und/oder Behandlung von Asthma bronchiale oder Heuschnupfen als Allergie vom Typ I nach Gell und Coombs und/oder Kontaktallergien als Allergien vom Typ II, III oder IV.

11. Verwendung nach einem der Ansprüche 1 bis 8 zur Unterdrückung von Transplantat-Abstoßungen bei Nieren- oder Knochenmarktransplantationen.

12. Pharmazeutische Zubereitung, umfassend einen Inhibitor oder mehrere Inhibitoren von Alanyl-Aminopeptidasen und/oder Enzyme gleicher Substratspezifität und Peptidfragmente von pathogenen Autoantigenen oder synthetische Analoga und/oder spezifische antigene Komponenten pathogener Mikroorganismen sowie gegebenenfalls einen oder mehrere pharmakologisch unbedenkliche Träger-, Zusatz- und/oder Hilfsstoffe.

13. Pharmazeutische Zubereitung nach Anspruch 12, umfassend als einen Inhibitor oder mehrere Inhibitoren von Alanyl-Aminopeptidasen und/oder Enzyme gleicher Substratspezifität mindestens ein Mitglied, gewählt aus der Gruppe, bestehend aus Actinonin, Leuhistin, Phebestin, Amastatin, Bestatin, Probestin, Arphamenin, MR 387, β-Aminothiole, α-Aminophosphinsäuren und deren Ester und Salze, α-Aminophosphonate, α-Aminoboronsäuren, α-Aminoaldehyde, Hydroxamate von α-Amino-säuren, N-Phenylphthalimide, N-Phenylhomophthalimide, α-Ketoamide, Thalidomid und dessen Derivate.

14. Pharmazeutische Zubereitung nach Anspruch 13, umfassend als einen Inhibitor oder mehrere Inhibitoren von Alanyl-Aminopeptidasen und/oder Enzyme gleicher Substratspezifität α-Ketoamide, 3-Amino-2-oxo-4-phenylbutansäureamide, α-Aminophosphinsäuren, D-Phe-γ[PO(OH)-CH₂]-Phe-Phe, N-Phenyl-homophthalimide, PAQ-22, α-Aminophosphonate, RB3014 und/oder Phebestin.

15. Pharmazeutische Zubereitung nach Anspruch 14, umfassend als einen Inhibitor oder mehrere Inhibitoren der Alanyl-Aminopeptidase und/oder von Enzymen gleicher Substratspezifität PAQ-22, RB3014 und/oder Phebestin.

16. Pharmazeutische Zubereitung nach einem der Ansprüche 12 bis 15, umfassend als Enzym gleicher Substratspezifität zytosolische Alanyl-Aminopeptidase.

17. Pharmazeutische Zubereitung nach Anspruch 16, umfassend als einen Inhibitor oder mehrere Inhibitoren eines Enzyms gleicher Substratspezifität PAQ-22.

18. Pharmazeutische Zubereitung nach einem der Ansprüche 12 bis 17, umfassend als Peptidfragmente von pathogenen Autoantigenen MBP (Myelin-Basisches-Protein), MOG (Myelin-Oligodendrozyten-Glykoprotein), MAG (Myelin-Assoziiertes-Glykoprotein) und/ oder PLP (Proteolipid-Protein).

19. Pharmazeutische Zubereitung nach einem der Ansprüche 12 bis 18, umfassend als spezifische antigene Komponenten pathogener Mikroorganismen Hüllproteine oder Membranglycolipid-Komplexe.
